# FASCICULE DE BREVET EUROPEEN

(11) **EP 1 862 195 B1**
(45) Date de publication et mention de la délivrance du brevet: **22.07.2009**
(21) Numéro de dépôt: 07290674.6
(22) Date de dépôt: 31.05.2007
(51) Int. Cl.: A61N 1/372, H03H 9/17

(54) **Dispositif médical actif tel qu'implant actif ou programmateur pour un tel implant, comprenant des moyens de télémétrie RF**
Aktive medizinische Vorrichtung wie beispielsweise aktives Implantat oder Programmiergerät für ein solches Implantat, umfassend RF-Telemetriemittel
Active medical device such as an active implant or programmer for such an implant, comprising RF telemetry means

(30) Priorité: 02.06.2006 FR 0604933
(43) Date de publication de la demande: 05.12.2007
(73) Titulaire: ELA MEDICAL, 92541 Montrouge (FR)
(72) Inventeur: Dal Molin, Renzo, 92320 Chatillon (FR)
(74) Mandataire: Dupuis-Latour, Dominique

(56) Documents cités:
- EP-A1- 1 484 083
- WO-A-01/78831
- US-A- 5 714 917
- US-A- 5 944 659
- US-A1- 2004 140 869
- US-A1- 2005 028 336
- US-B1- 6 659 947
- US-B2- 6 868 288

## Description

L'invention concerne les "dispositifs médicaux actifs" tels que définis par la directive 93/42/CE du 14 juin 1993 du Conseil des communautés européennes, et notamment les "dispositifs médicaux implantables actifs" tels que définis par la directive du Conseil 90/385/CEE du 20 juin 1990.

Cette définition inclut en particulier les appareils chargés de surveiller l'activité cardiaque et de générer des impulsions de stimulation, de resynchronisation, de défibrillation et/ou de cardioversion en cas de trouble du rythme détecté par l'appareil. Elle inclut aussi les appareils neurologiques, les pompes de diffusion de substances médicales, les implants cochléaires, les capteurs biologiques implantés, etc., ainsi que les dispositifs de mesure de pH ou encore d'impédance intracorporelle (telle que mesure d'impédance transpulmonaire ou d'impédance intracardiaque). On notera également que, si l'invention s'applique de manière particulièrement avantageuse aux appareils implantés tels que stimulateurs, cardioverteurs ou défibrillateurs, elle peut tout aussi bien être mise en oeuvre avec des dispositifs médicaux non implantés, par exemple des enregistreurs de données comme les appareils Holter externes destinés à la surveillance et à l'enregistrement en ambulatoire de certains paramètres physiologiques tels que par exemple l'activité cardiaque.

De façon générale, la plupart des dispositifs médicaux actifs sont conçus pour permettre un échange de données avec un "programmateur", qui est un appareil externe permettant de vérifier le paramétrage du dispositif, de lire des informations enregistrées par celui-ci ou d'y inscrire des informations, ou encore de mettre à jour le logiciel interne de pilotage du dispositif.

Cet échange de données entre le dispositif médical et le programmateur est effectué par télémétrie, c'est-à-dire par une technique de transmission à distance d'informations, sans contact galvanique.

Jusqu'à présent, la télémétrie est opérée par couplage essentiellement inductif entre des bobines du dispositif implanté et du programmateur, technique connue sous le nom de "procédé par induction". Cette technique présente cependant l'inconvénient, en raison de la très faible portée d'un couplage inductif, de nécessiter l'utilisation d'une "tête de télémétrie" reliée au programmateur et contenant une bobine qu'un opérateur place au voisinage du site où est implanté le dispositif.

Il a été récemment proposé de mettre en oeuvre une autre technique de couplage non galvanique, utilisant les deux composantes d'une onde électromagnétique produite par des circuits émetteurs/récepteurs opérant dans le domaine des radiofréquences (RF), typiquement des fréquences de l'ordre de plusieurs centaines de mégahertz.

Cette technique, dite de "télémétrie RF" permet de programmer ou interroger des implants à des distances supérieures à 3 m, et autorise donc l'échange d'informations sans manipulation d'une tête de télémétrie, voire même sans intervention d'un opérateur externe.

Un dispositif comprenant des moyens de télémétrie RF, et son programmateur associé, sont par exemple décrits dans le US-A-6 868 288 (Thompson).

Le fonctionnement satisfaisant des circuits de télémétrie RF implique une élimination efficace des parasites RF susceptibles de produire des interférences et de perturber la transmission des données. En effet, à la différence des techniques par induction, qui présentent une bonne immunité aux parasites, la réception des signaux RF est fortement perturbée par l'environnement électromagnétique, notamment les signaux de radio, de télévision et de téléphonie mobile, sans compter les nombreux parasites industriels susceptibles d'être produits dans l'environnement immédiat du porteur de l'implant.

Les circuits de télémétrie RF nécessitent donc l'utilisation de filtres passe-bande très efficaces, présentant une caractéristique de réjection de bande très abrupte.

Les filtres à ondes acoustiques présentent de telles propriétés, et le US-A-6 868 288 précité propose précisément d'utiliser dans le circuit de télémétrie RF d'un implant un résonateur à ondes acoustiques de surface SAW (*Surface Acoustic Wave)* ou un résonateur acoustique de volume à film mince FBAR *(Thin-Film Bulk Acoustic Resonator).* Les propriétés de très grande sélectivité de ces résonateurs sont en effet connues, par ailleurs, pour la réalisation de filtres passe-bande très efficaces.

Ces filtres à résonateur SAW ou FBAR présentent cependant un certain nombre d'inconvénients, tout particulièrement lorsqu'ils sont utilisés dans des dispositifs implantés.

Ainsi, les résonateurs SAW utilisent, par principe, la propagation d'une onde acoustique en surface, qui implique corrélativement une taille de composant relativement importante. Cet inconvénient est particulièrement handicapant avec les dispositifs implantés qui, comme on le comprend aisément, requièrent une miniaturisation poussée du circuit électronique compte tenu de la place réduite disponible à l'intérieur du boîtier.

D'autre part, du point de vue de la technologie, ces résonateurs SAW sont disponibles seulement sous forme de composants discrets, qui doivent donc être reportés sur le circuit électronique de l'implant, avec les inconvénients qui en découlent en termes notamment d'étapes de procédé supplémentaires et de coût supérieur.

Enfin, sur le plan électrique, les résonateurs SAW présentent une excellente sélectivité mais introduisent des pertes d'insertion non négligeables dans les circuits où ils sont utilisés, dégradant d'autant la sensibilité du récepteur de télémétrie.

A la différence des résonateurs SAW, les résonateurs FBAR présentent une taille beaucoup plus réduite et des pertes d'insertion moindres.

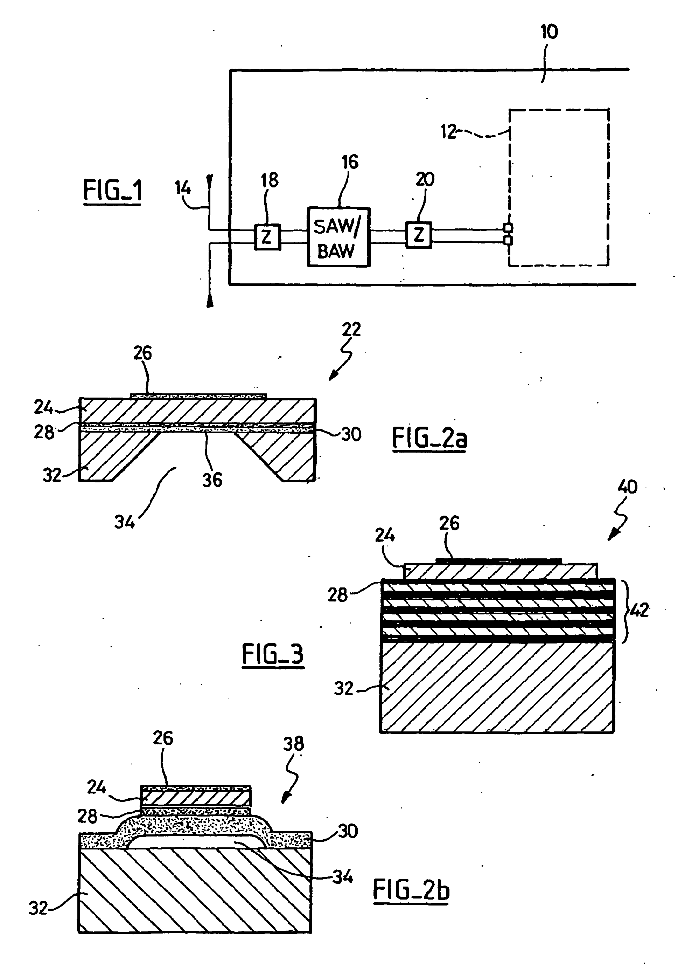

En revanche, ces résonateurs FBAR sont beaucoup plus délicats à réaliser, car ils nécessitent le micro-usinage d'une très fine membrane mobile, susceptible d'être mise en résonance (on décrira plus en détail la structure des résonateurs FBAR en référence aux figures 2a et 2b). Ce micro-usinage est délicat à mettre en oeuvre, notamment dans un processus collectif, ce qui introduit de nombreux rebuts lors de la fabrication des composants.

De plus, le mouvement de la membrane mobile implique de laisser subsister un espace libre au-dessus et au-dessous de celle-ci. De ce fait, si des poussières venaient à se déposer au-dessus de la membrane en cours de fabrication ou postérieurement, la fréquence de résonance de la membrane se trouverait modifiée, et il serait nécessaire de réaccorder le filtre. Pour pallier cet inconvénient, une fois réalisé et ajusté en fréquence, le composant doit être enfermé dans un volume étanche, avec formation d'une couche supplémentaire ou report d'un capot de fermeture du composant.

Du point de vue industriel, bien qu'il soit théoriquement possible d'intégrer le résonateur FBAR au cours du processus de fabrication du circuit auquel il est associé, une telle intégration se révèle difficile à mettre en oeuvre avec un rendement satisfaisant, de sorte que les résonateurs FBAR aujourd'hui disponibles le sont seulement sous forme de composants discrets. Le composant FBAR doit donc être reporté sur le circuit électronique ou sur la plaquette hybride par un procédé additionnel tel que *wire-bonding* ou *flip-chip,* ce qui, comme dans le cas du SAW, grève le coût de fabrication et réduit les performances électriques du fait de la présence des fils, pistes de liaison, etc.

Enfin, outre la difficulté qu'il y a à trouver et maintenir l'accord d'un composant résonateur FBAR, celui-ci présente une caractéristique de réjection moins abrupte que celle d'un résonateur SAW, de sorte que pour obtenir une réjection satisfaisante, il est nécessaire de combiner entre eux plusieurs résonateurs FBAR. Cette multiplication des composants augmente d'autant le coût, l'encombrement, et les difficultés de mise au point du circuit final.

Un premier but de l'invention est de réaliser un dispositif médical actif, et/ou son programmateur associé, comprenant des circuits de télémétrie RF dépourvus de résonateurs FBAR pour remédier aux inconvénients ci-dessus, et qui soient adaptés aux contraintes particulières des implants actifs qui requièrent une miniaturisation plus poussée du circuit électronique compte tenu de la place réduite disponible à l'intérieur du boîtier de l'implant.

L'invention a notamment pour but de réaliser un tel dispositif et/ou programmateur dont les circuits RF présentent les avantages suivants :
- coût de fabrication réduit ;
- filtre éventuellement intégrable, de manière à pouvoir obtenir un composant monolithique incluant la puce du circuit émetteur/récepteur RF avec son filtre réjecteur de bande associé. Une telle intégration présente de nombreux avantages : réduction du coût d'ensemble, diminution des pertes d'insertion et maximisation de la surface utile (pour miniaturiser les implants, on sait aujourd'hui empiler plusieurs puces monolithiques les unes sur les autres ;
- réalisation collective possible avec un bon rendement du processus de fabrication (moindres rebuts) ;
- réduction des impédances parasites, notamment des inductances (conducteurs de liaison, pistes, *bondings,* etc.), permettant de réduire les pertes d'insertion et éviter d'avoir à réaccorder le filtre après intégration ou report sur le circuit ;
- possibilité d'utilisation en nombre des résonateurs, permettant de concevoir des filtres performants combinant plusieurs résonateurs pour obtenir une caractéristique très abrupte.

Un autre but de l'invention est, surtout, de proposer un dispositif actif comportant des circuits de télémétrie RF susceptibles de fonctionner dans plusieurs bandes de fréquences, telles que la bande MICS *(Medical Implants Communication System)* 402-405 MHz, ou les bandes banalisées publiques ISM (Industriel, Scientifique et Médical) 863-870 MHz, 902-928 MHz et 2,4 GHz utilisées par les dispositifs médicaux, ou encore une transmission de type *Ultra Wide Band* UWB, qui est une technique où les signaux émis sont des impulsions de Dirac produisant dans le domaine fréquentiel un très large spectre (donc sans filtrage dans ce dernier cas).

En effet, les appareils dotés de fonctions de télémétrie RF actuels sont tous des appareils seulement multicanaux, c'est-à-dire utilisant plusieurs fréquences situées dans une même bande.

Il est certes connu, dans le domaine médical, d'assurer la transmission de données dans des bandes differentes, comme décrit par exemple dans le US 6 659 947 B1. Mais cette technique concerne les architectures de réseaux locaux sans fil (WLAN), où chaque bande est spécialisée dans la transmission d'un type de données particulier : par exemple données de télémétrie dans la bande 608-614 MHz (communication avec des implants), et données générales dans la bande 2,4 GHz (accès aux bases de données de l'hôpital).

Or il serait particulièrement avantageux de disposer, pour la transmission des signaux de télémétrie RF, d'un appareil multi-bande (bibande, tribande, voire même quadribande), car :
- les bandes de fréquences autorisées ne sont pas forcément les mêmes selon les pays ;
- selon les circonstances, la propagation peut être meilleure dans telle ou telle bande, et il serait avantageux de pouvoir choisir celle qui assure la meilleure propagation de manière à optimiser la transmission des données ;
- l'occupation de la bande (le nombre de canaux utilisés) varie selon les bandes et, là encore, il pourrait être avantageux de choisir une bande présentant suffisamment de canaux libres.

Le problème de l'invention est résolu, et les différents buts précités sont atteints, par un dispositif médical actif, ou son programmateur associé, du type général divulgué par le US-A-6 868 288 comprenant les caractéristiques énoncées par la revendication 1.

Une structure de résonateur BAW à ondes acoustiques de volume du type SMR à isolement par miroir de Bragg acoustique est décrite dans le US-A-2004/0140869. Mais ce document propose seulement d'utiliser le composant pour ses performances électriques supérieures, notamment en termes de facteur de surtension (facteur Q) et de coefficient de couplage, afin de concevoir des filtres dont la caractéristique présente des fronts plus raides.

Les sous-revendications énoncent des formes de réalisation avantageuses.

On va maintenant décrire un exemple de réalisation de l'invention, en référence aux dessins annexés où les mêmes références numériques désignent d'une figure à l'autre des éléments identiques ou fonctionnellement semblables.

La figure 1 illustre, de façon schématique, un circuit émetteur/récepteur de télémétrie RF couplé à son antenne associée, dans le cas d'un dispositif de l'art antérieur.

Les figures 2a et 2b illustrent la structure de principe d'un résonateur de type FBAR à membrane micro-usinée, selon les deux variantes respectives à membrane supportée ou à cavité.

La figure 3 illustre la configuration générale d'un résonateur de type SMR à réflecteur acoustique de Bragg.

La figure 4 illustre les circuits émetteurs/récepteurs de télémétrie RF et la manière dont ils sont couplés à une antenne commune, dans un dispositif actif de type bi-bande selon l'invention.

La figure 5 illustre de façon plus détaillée la structure d'un résonateur BAW de type SMR à miroir de Bragg acoustique.

La figure 6 illustre la structure d'un filtre réjecteur asymétrique réalisé à partir d'une combinaison de résonateurs montés en échelle.

La figure 7 montre la structure d'un filtre réjecteur symétrique, réalisé à partir d'une combinaison de résonateurs montés en treillis.

Les figures 8, 9 et 10 illustrent trois techniques possibles d'intégration ou de report des résonateurs SMR sur un circuit de télémétrie RF selon l'invention.

Sur la figure 1, on a représenté de façon schématique la configuration d'un circuit de télémétrie RF d'un dispositif médical actif selon la technique antérieure.

Le circuit principal 10, typiquement réalisé sur un substrat hybride, comprend un circuit émetteur/récepteur de télémétrie RF 12 couplé à une antenne 14 incorporée au dispositif (par exemple dans la région de la tête de connexion de l'implant).

La réjection des signaux parasites est assurée par interposition entre l'antenne 14 et les circuits 12 d'un résonateur 16 tel qu'un résonateur à onde acoustique de surface SAW ou à onde acoustique de volume BAW 16. Des composants d'adaptation d'impédance 18, 20 sont prévus en tant que de besoin pour assurer le couplage et la symétrisation éventuelle ainsi que pour réduire les pertes d'insertion.

Lorsque les filtres de réjection des circuits de télémétrie RF des implants connus mettent en oeuvre des résonateurs BAW, ceux-ci sont des composants de type FBAR présentant l'une des deux structures illustrées figure 2a et 2b.

Dans la première structure, illustrée figure 2a, le composant 22 comprend une couche 24 de matériau piézoélectrique excité par des électrodes conductrices 26, 28. Lorsqu'il est sollicité, ce matériau transmet une fraction de son énergie à une membrane 30 en matériau non piézoélectrique, micro-usinée sur un substrat massif 32. D'autre part, une partie 34 de ce substrat massif 32 a été éliminée localement, de manière à former une cavité 34 qui pourra être excitée par la vibration de la partie apparente 36 de la membrane.

Dans la seconde variante, illustrée figure 2a, le substrat 32 est conservé intact, et la cavité 34 est formée en réservant un intervalle d'air entre le résonateur 24 et le substrat 32. Cet intervalle est par exemple obtenu en déposant une couche sacrificielle entre résonateur et substrat de sorte qu'une fois cette couche enlevée le résonateur 24 se trouve suspendu au-dessus du substrat.

Comme on le comprend aisément, ces structures sont délicates à réaliser et fragiles. De plus, l'ajustement de la fréquence de résonance du composant dépend très fortement des propriétés du matériau piézoélectrique, de l'épaisseur des couches, du dimensionnement de la cavité résonante et des diverses contraintes mécaniques exercées sur la membrane, qui a tendance à se déformer et à se déchirer aisément.

Le point de départ de l'invention consiste à choisir pour le filtre de réjection de bande une structure de résonateur autre que celle du résonateur FBAR illustrée sur les figures 2a et 2b.

Plus précisément, l'invention propose d'utiliser, dans un circuit de télémétrie RF pour dispositif médical actif, notamment pour un implant actif, un résonateur de type SMR (*Solidly Mounted Resonator*), qui est une configuration différente de résonateur piézoélectrique, liée au substrat et ne mettant en oeuvre aucune cavité ni membrane.

La structure générale d'un composant de type SMR est illustrée figure 3. Ce composant est constitué d'un résonateur 40 comprenant une couche 24 de matériau piézoélectrique, par exemple du nitrure d'aluminium AIN, disposée entre deux couches métalliques 26, 28 formant électrodes d'excitation, par exemple en molybdène.

Pour éviter que l'onde acoustique ne s'y propage et ne s'y atténue, la structure résonante est acoustiquement isolée du substrat 32 par une interface composée d'un empilement 42 de couches quart d'onde d'impédances acoustiques très différentes, alternativement élevées et faibles, permettant de réaliser un miroir de Bragg acoustique. Les couches constituant l'empilement 42 sont par exemple des couches alternées de nitrure de silicium SiN et oxycarbure de silicium SiOC, et leur nombre peut atteindre dix ou douze, voire plus. Pour plus de détails, on pourra se référer au US-A-2004/0140869 précité, qui expose la théorie et les performances électriques d'un tel composant.

Ce résonateur présente l'avantage de pouvoir être réalisé sur une très grande variété de substrats (les propriétés acoustiques du substrat étant sans importance) et d'être également intégrable au processus global de fabrication du circuit monolithique auquel sera associé le résonateur.

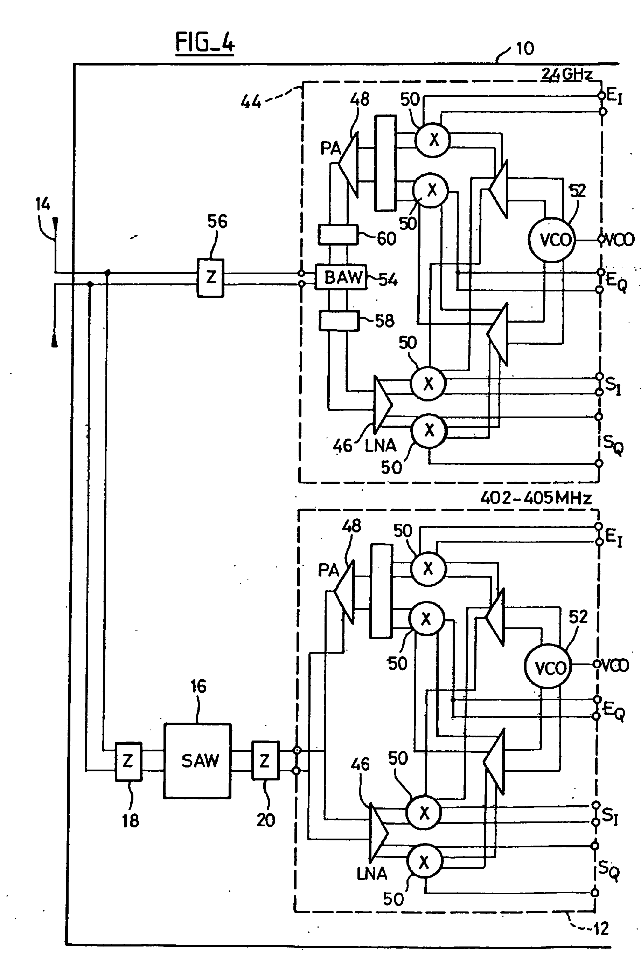

La figure 4 illustre la configuration d'un circuit de télémétrie RF bibande utilisant un tel résonateur SMR, par exemple un circuit de télémétrie RF susceptible de fonctionner indifféremment dans les bandes 402-405 MHz et 2,4 GHz.

Le circuit de télémétrie RF 10 comprend deux étages émetteur/récepteur respectifs 12, 44 fonctionnant dans chacune des deux bandes concernées. Ces circuits présentent une structure semblable, avec côté réception un amplificateur faible bruit LNA 46 et côté émission un amplificateur de puissance PA 48. Ces amplificateurs de réception et d'émission 46, 48 sont couplés à divers étages mélangeurs 50 et à un oscillateur commandé en tension VCO 52. L'ensemble est contrôlé par des signaux d'entrée de données en phase E_{I} et en quadrature E_{Q} et par un signal de commande de la fréquence de l'oscillateur VCO, et délivre en sortie des signaux de données en phase Sₗ et en quadrature S_{Q}. Les deux circuits d'émission/réception 12, 44 sont couplés à une antenne commune 14, par exemple un doublet incorporé au boîtier de l'implant et relié par une ligne symétrique bifilaire à chacun des deux circuits d'émission/réception.

Le circuit 12 opérant dans la bande 402-405 MHz est, de manière en elle-même connue, couplé à l'antenne 14 par l'intermédiaire d'un filtre réjecteur 16 comprenant un résonateur à ondes acoustiques de surface SAW, avec des éléments appropriés 18, 20 d'adaptation d'impédance. Le circuit 44 opérant dans la bande 2,4 GHz est couplé à l'antenne 14 par l'intermédiaire d'un filtre réjecteur 54 comprenant un résonateur BAW de type SMR, ou une combinaison de plusieurs résonateurs de ce type (voir plus bas). Le couplage du filtre 54 au reste du circuit est assuré par des éléments respectifs référencés 56 côté antenne, 58 côté réception et 60 côté émission.

La liaison de télémétrie RF peut être établie indifféremment dans la bande 402-405 MHz ou dans la bande 2,4 GHz. Le choix de l'une ou l'autre bande pour la transmission des données peut être avantageusement effectué de façon automatique en fonction d'un ou plusieurs critères dont, notamment :
- le niveau de signal capté en réception RSSI (*Received Signal Strength Indicator*) des signaux RF reçus, dans l'une et l'autre bande, par l'implant (dans le sens de transmission programmateur → implant) et par le programmateur (dans le sens de transmission implant → programmateur)
- le débit maximal des données susceptibles d'être transmis dans l'une et l'autre bande, et/ou
- l'occupation des différents canaux dans l'une et l'autre bande, de manière à s'assurer de pouvoir disposer d'un canal de communication libre.

Chaque fois qu'une communication de télémétrie RF est initiée, la recherche de la meilleure bande est opérée, et la transmission des données est effectuée sur la bande choisie. Avantageusement, les circuits électroniques de la bande qui n'a pas été choisie sont mis dans un mode de faible consommation, de manière à réduire d'autant l'énergie consommée par la pile de l'implant.

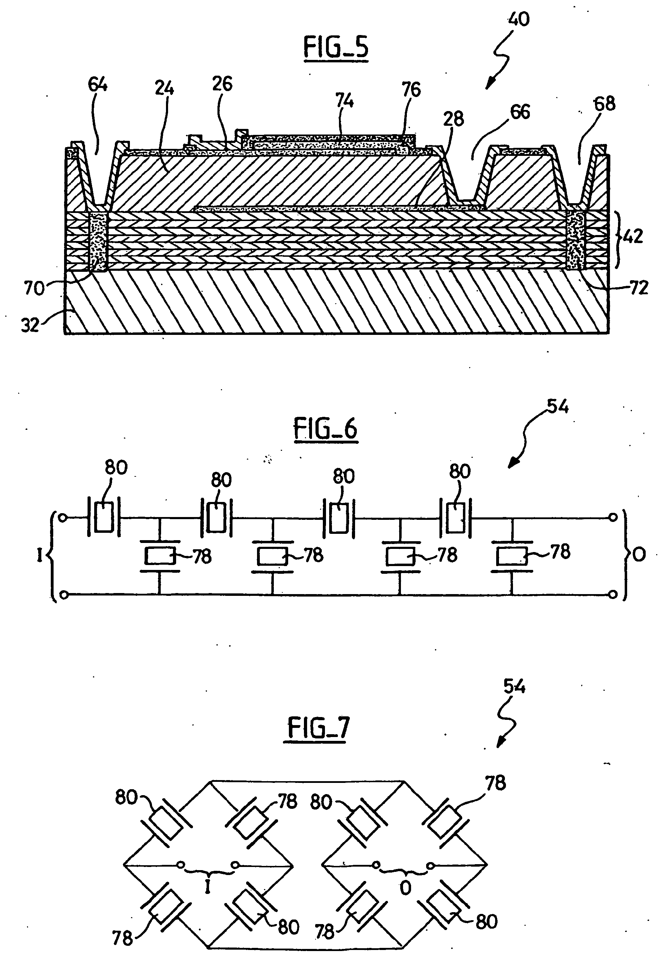

La figure 5 décrit plus en détail le résonateur BAW de type SMR utilisé (seul ou en combinaison) comme filtre de réjection de bande pour le dispositif de l'invention.

Très avantageusement, ce résonateur est formé au cours du même processus que le circuit émetteur/récepteur de télémétrie RF, c'est-à-dire que la puce RF est une puce monolithique intégrant son propre résonateur SMR.

Comme on l'avait expliqué plus haut en référence à la figure 3, le résonateur 40 comprend une couche piézoélectrique 24, par exemple en nitrure d'aluminium AIN, disposée entre deux électrodes métalliques d'excitation 26, 28, par exemple en molybdène. Lorsqu'un signal RF est appliqué entre ces deux électrodes, une déformation mécanique est créée dans le matériau et, pour une fréquence prédéterminée, les ondes viennent se renforcer et le composant entre en résonance. Le résonateur est caractérisé par sa fréquence propre, son coefficient de couplage (représentatif des pertes d'insertion) et son facteur de qualité. La fréquence propre est déterminée par divers paramètres tels que la vitesse acoustique dans le matériau, son module d'Young et l'épaisseur des diverses couches du composant. Du fait du comportement mécanique du résonateur, celui-ci doit être isolé du substrat 32 sur lequel il est formé de manière que la résonance puisse s'établir avec un bon coefficient de couplage. Dans le cas d'un résonateur de type SMR, cet isolement est réalisé par un empilement 42 de couches SiN/SiOC formant réflecteur de Bragg acoustique. L'épaisseur de chaque couche dépend de la fréquence propre du résonateur (quart d'onde) et le nombre de couches est calculé pour optimiser la réflexion finale, typiquement au moins 99% du signal acoustique étant réfléchi. Pour assurer la liaison électrique entre les électrodes d'excitation du cristal piézoélectrique et les différents circuits de la puce, la couche 24 est creusée, par exemple en 64, 66, 68, et revêtue d'une métallisation, jusqu'à atteindre des vias tels que 70, 72 formés au travers de l'empilement 42 formant miroir de Bragg, dans des régions d'extrémité situées au-delà de la région de la couche piézoélectrique 24 entraînée en vibration par la sollicitation électrique des électrodes 26, 28.

Très avantageusement, le résonateur 40 comprend à sa surface supérieure une couche de charge 74, par exemple en SiO₂, qui est neutre sur le plan électrique mais qui a pour effet, du fait de sa masse, d'influer sur la fréquence propre de la structure résonante. Cette couche permet, par un enlèvement sélectif contrôlé de matière (par exemple érosion par laser ou par faisceau d'ions) d'enlever progressivement une quantité réduite de matière jusqu'à obtenir la fréquence précise de résonance souhaitée *(trimming).* Une couche de passivation 76 de nitrure de silicium SiN) protège la structure du résonateur une fois celui-ci accordé sur la fréquence propre choisie.

Les figures 6 et 7 illustrent deux exemples de combinaisons de résonateurs BAW de type SMR tel que celui que l'on vient de décrire, permettant d'obtenir un filtre de réjection de bande présentant les performances souhaitées.

La figure 6 illustre un filtre de réjection de bande 54 avec entrée/sortie I/O asymétrique, les résonateurs étant associés suivant un montage en échelle, par exemple huit résonateurs, avec quatre résonateurs parallèle 78 et quatre résonateurs série 80. Les résonateurs série et parallèle ont des fréquences de résonance légèrement décalées, la bande passante du filtre 54 étant liée à ce décalage, avec une fréquence de résonance des résonateurs parallèle correspondant à la fréquence d'antirésonance des résonateurs série pour minimiser les pertes d'insertion.

La figure 7 illustre une autre combinaison possible de quatre résonateurs parallèle 78 et quatre résonateurs série 80 dans une configuration en treillis, adaptée à la réalisation d'un filtre symétrique, pour des circuits différentiels.

Le choix d'un SMR comme résonateur permet, compte tenu du rendement technologique élevé (peu de rebuts), de réaliser de façon monolithique un filtre de réjection de bande comprenant une pluralité de résonateurs associés, typiquement un filtre à quatre ou huit résonateurs. Compte tenu de la multiplication des résonateurs dans un même filtre, un taux de rebut trop élevé par résonateur (comme cela est le cas des FBAR) conduirait en effet à un déchet considérable pour un filtre comportant quatre ou huit de ces résonateurs, excluant une intégration poussée.

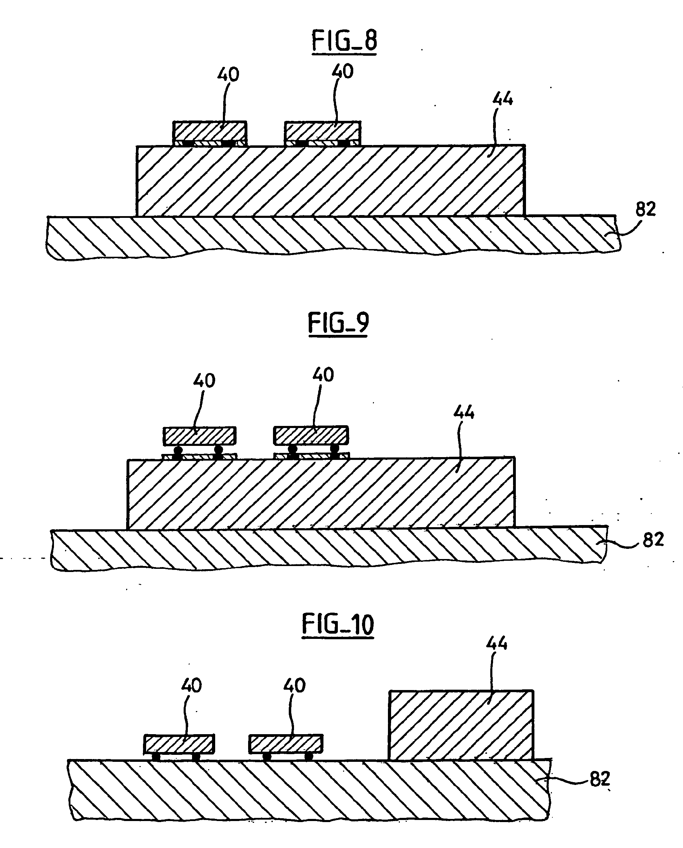

Les figures 8 à 10 illustrent plusieurs solutions technologiques pour associer des résonateurs 40, notamment combinés à plusieurs pour réaliser un filtre de réjection de bande, à la puce monolithique 44 du circuit émetteur/récepteur de télémétrie RF, cette puce étant elle-même reportée sur une plaquette de circuit hybride 82 supportant et interconnectant l'ensemble des composants, actifs et passifs, du dispositif médical.

La première solution, illustrée figure 8, consiste à réaliser les divers résonateurs 40 directement en surface de la puce 44, de manière intégrée au cours de la même étape de processus. Pour plus de détails sur cette technique d'intégration, on pourra se référer au FR-A-2 853 473, qui décrit un composant électronique incorporant un ou plusieurs résonateurs associés, réalisés de manière monolithique.

La deuxième solution, illustrée figure 9, consiste à réaliser les résonateurs sous forme de composants discrets autonomes, puis à les reporter sur la puce 44, par exemple par une technique de type *flip-chip* ou analogue. Avec cette solution, le rendement de fabrication peut être augmenté par un tri préalable des résonateurs avant leur report sur la puce 44.

La troisième solution, illustrée figure 10, consiste à réaliser également les résonateurs 40 sous forme de composants discrets, et à les reporter non plus sur la puce 44, mais sur la plaquette de circuit hybride 82 recevant, par ailleurs, la puce de circuit 44.

On notera que les deux premières techniques sont compatibles avec un empilement des puces monolithiques, où la puce des circuits émetteurs/récepteurs de télémétrie RF est par exemple empilée sur la puce du microcontrôleur de traitement des signaux, au lieu d'être disposée à côté de celle-ci. Cette configuration présente l'avantage d'économiser la place disponible pour les circuits électroniques à l'intérieur du boîtier de l'implant. L'intégration des résonateurs au-dessus de la puce de télémétrie RF, elle-même superposée à la puce du microcontrôleur, n'implique qu'une augmentation d'épaisseur globale de l'empilement de quelques dizaines de micromètres supplémentaires, négligeable sur le plan de l'encombrement.

Un autre avantage de cette configuration empilée est qu'elle permet de réduire fortement les impédances, notamment les inductances réparties des conducteurs de liaison, pistes, *bondings,* etc. et évite d'avoir à réaccorder le résonateur du filtre de réjection (ou les différents résonateurs combinés pour former ce filtre de réjection) après report sur la puce.

## Revendications

1. Un dispositif médical actif de type implant actif ou programmateur pour un tel implant, comprenant :
- au moins une antenne RF (14), et
- au moins un émetteur/récepteur de télémétrie RF (44) avec, pour le couplage à l'antenne, un filtre de réjection de bande (54) associé,
**caractérisé en ce que** le dispositif comprend une pluralité d'émetteurs/récepteurs de télémétrie RF (12 ; 44) opérant dans des bandes de fréquences respectives distinctes, avec pour chaque émetteur/récepteur un filtre de réjection de bande respectif (16 ; 54), au moins l'un (54) de ces filtre de réjection de bande incluant au moins un résonateur BAW à ondes acoustiques de volume du type SMR à isolement par miroir de Bragg acoustique.

2. Le dispositif de la revendication 1, dans lequel le dispositif comprend une antenne (14) commune aux émetteurs/récepteurs de télémétrie RF (12 ; 44) et couplée à ces derniers par l'intermédiaire desdits filtres de réjection de bande (16 ; 54) et d'éléments d'adaptation d'impédance (18, 20 ; 56, 58, 60).

3. Le dispositif de la revendication 1, dans lequel les émetteurs/récepteurs de télémétrie RF opèrent dans au moins deux bandes de fréquences parmi les bandes : 402-405 MHz, 863-870 MHz, 902-928 MHz et 2,4 GHz ou par transmission de type UWB.

4. Le dispositif de la revendication 1, dans lequel le dispositif comprend des moyens d'évaluation de la performance des différentes bandes de fréquences, et des moyens de sélection, pour la transmission des signaux de télémétrie RF, de celui des émetteurs/récepteurs opérant dans celle de ces bandes présentant la meilleure performance.

5. Le dispositif de la revendication 4, dans lequel les moyens d'évaluation de la performance sont des moyens aptes à évaluer des critères parmi : le niveau de signal capté en réception, le débit maximal de données susceptible d'être transmis, et l'occupation des différents canaux dans les bandes respectives.

6. Le dispositif de la revendication 4, dans lequel le dispositif comprend des moyens pour commuter en un mode de faible consommation l'(les) émetteur(s)/récepteur(s) opérant dans la(les) bande(s) non sélectionnée(s).

7. Le dispositif de la revendication 1, dans lequel :
- le dispositif comprend une plaquette de circuit hybride (82) et au moins une puce, reportée sur cette plaquette, de circuit monolithique intégrant le(s)dit(s) émetteur(s)/récepteur(s) de télémétrie RF (44), et
- le résonateur BAW (40) est directement intégré à ladite puce en surface de celle-ci.

8. Le dispositif de la revendication 1, dans lequel :
- le dispositif comprend une plaquette de circuit hybride (82) et au moins une puce, reportée sur cette plaquette, de circuit monolithique intégrant le(s)dit(s) émetteur(s)/récepteur(s) de télémétrie RF (44), et
- le résonateur BAW (40) est un composant discret, distinct de la puce et reporté en surface de celle-ci.

9. Le dispositif de la revendication 1, dans lequel :
- le dispositif comprend une plaquette de circuit hybride (82) et au moins une puce, reportée sur cette plaquette, de circuit monolithique intégrant le(s)dit(s) émetteur(s)/récepteur(s) de télémétrie RF (44), et
- le résonateur BAW (40) est un composant discret, distinct de la puce et reporté en surface de la plaquette de circuit hybride (82).

10. Le dispositif de la revendication 1, dans lequel le résonateur BAW (40) est un résonateur accordable comprenant une couche superficielle de charge érodable (74).

11. Le dispositif de la revendication 1, dans lequel le filtre de réjection de bande (54) est un filtre à entrée et sortie différentielles comprenant une pluralité de résonateurs BAW (78, 80) configurés en treillis.

12. Le dispositif de la revendication 1, dans lequel le filtre de réjection de bande (54) est un filtre à entrée et sortie asymétriques comprenant une pluralité de résonateurs BAW (78, 80) configurés en échelle.

## Claims

1. An active implant-type active medical device or programmer for such implant, comprising :
- at least one antenna (14) and
- at least one RF telemetry transceiver (44) with an associated band rejection filter (54) for coupling to the antenna,
**characterized in that** said device comprises a plurality of RF telemetry transceivers (12 ; 44) working in distinct respective frequency bands, with for each transceiver one respective band rejection filter (16 ; 54), wherein at least one (54) of said band rejection filters includes at least one bulk acoustic wave BAW solidly mounted resonator insulated by an acoustic Bragg mirror.

2. The device according to Claim 1, wherein said device comprises an antenna (14) that is common to said RD telemetry transceivers (12 ; 44) and coupled to the latter through said band rejection filters (16 ; 54) and impedance adaptation elements (20 ; 56, 58, 60).

3. The device according Claim 1, wherein said RF telemetry transceivers work in at least two frequency bands chosen between following bands : 402-405 MHz, 863-870 MHz, 902-928 MHz and 2.4 GHz of through UWB-type transmission.

4. The device according to Claim 1, wherein said device comprises means for evaluating the performance of different frequency bands and means for selecting, for transmission of RF telemetry signals, that transceiver working in that band exhibiting the best performance.

5. The device according to Claim 4, wherein said means for evaluating the performance are means adapted to evaluate criteria such as the level of received signals, the maximum data rate that might be transmitted, and the occupancy of the different channels in the respective bands.

6. The device according Claim 4, wherein said device comprises means for switching into a low consumption mode said transceiver(s) working in said non-selected band(s).

7. The device according to Claim 1, wherein :
- the device comprises a hybrid circuit wafer (82) and at least one monolithic circuit chip mounted onto said wafer and integrating said RF telemetry transceiver(s) (44) and
- said BAW resonator (40) is directly integrated and surface mounted on said chip.

8. The device according to Claim 1, wherein :
- the device comprises a hybrid circuit wafer (82) and at least one monolithic circuit chip mounted onto said wafer and integrating said RF telemetry transceiver(s) (44) and
- said BAW resonator (40) is a discrete component, distinct from said chip and surface mounted on it.

9. The device according to Claim 1, wherein :
- the device comprises a hybrid circuit wafer (82) and at least one monolithic circuit chip mounted onto said wafer and integrating said RF telemetry transceiver(s) (44) and
- said BAW resonator (40) is a discrete component, distinct from said chip and surface mounted on the hybrid circuit wafer (82).

10. The device according to Claim 1, wherein the BAW resonator (40) is a tunable resonator comprising an erodible surface load layer.

11. The device according to Claim 1, wherein the band rejection filter (54) is a filter with differential inlets/outlets comprising a plurality of BAW resonators (78, 80) connected in a lattice network.

12. The device according to Claim 1, wherein the band rejection filter (54) is a filter with differential inlets/outlets comprising a plurality of BAW resonators (78, 80) connected in a ladder network.

## Patentansprüche

1. Eine medizinische Vorrichtung des Typs aktives Implantat oder programmierbares Gerät für ein solches Implantat, umfassend :
- wenigstens eine RF-Antenne (14) und
- wenigstens einen RF-Telemetrie-Sender/Empfänger (44) mit einer zugehörigen, für eine Kopplung zur Antenne bestimmten Bandsperre (54),
**dadurch gekennzeichnet, dass** die Vorrichtung eine Vielzahl von in verschiedenen respektiven Frequenzbändern arbeitenden RF-Telemetrie-Sendern/Empfängern (12 ; 44) aufweist, wobei wenigstens eine (54) jener Bandsperren wenigstens einen mit akustischen Wellen arbeitenden, mit einem akustischen Bragg-Spiegel isolierten BAW Resonator des Typs SMR einschliesst.

2. Die Vorrichtung gemäss Anspruch 1, wobei die Vorrichtung eine mit den RF-Telemetrie-Sendern/Empfängern (12 ; 44) gemeinsame, mit letzeren durch die besagten Bandsperren (16 ; 54) und Impedanzanpassungselemente (18, 20 ; 56, 58, 60) gekoppelte Antenne (14) aufweist.

3. Die Vorrichtung gemäss Anspruch 12, in welcher die RF-Telemetrie-Sender/Empfänger in wenigstens zwei zwischen folgenden Bändern 402-405 MHz, 863-870 MHz, 902-928 MHz und 2,4 GHz ausgewählten Frequenzbändern oder durch UWB-Typ-Übertragung arbeiten.

4. Die Vorrichtung gemäss Anspruch 1, wobei die Vorrichtung Mittel zur Abschätzung der Leistungsfähigkeit der verschiedenen Frequenzbändern und zur Übertragung der RF-Telemetrie-Signale geeignete Mittel zum Auswahl desjenigen in demjenigen jener Bänder, das die beste Leistungsfähigkeit aufweist, arbeitenden Sender/Empfänger aufweist.

5. Die Vorrichtung gemäss Anspruch 4, in welcher die Mittel zur Abschätzung der Leistungsfähigkeit Mittel sind, die geeignet sind, zwischen der aufgefangenen Signalhöhe, der maximalen übertragbaren Datenrate und der Belegung der verschiedenen Kanäle in den respektiven Bändern ausgewählten Kriterien abzuschätzen.

6. Die Vorrichtung gemäss Anspruch 4, wobei die Vorrichtung Mittel aufweist, die geeignet sind, in einen Modus mit geringem Verbrauch das(die) in dem(den) nicht ausgewählte(n) Band(Bändern) arbeitende(n) Sender(n)/Empfänger(n) umzuschalten.

7. Die Vorrichtung gemäss Anspruch 1, wobei :
- die Vorrichtung ein Hybridschaltung-Plättchen (82) und wenigstens einen auf diesem Plättchen angebrachten Chip eines den(die) besagten RF-Telemetrie-Sender/Empfänger (44) integrierenden monolithischen Schaltkreises aufweist, und
- der BAW-Resonator (40) unmittelbar zu jenem Chip oberflächenmontiert integriert ist.

8. Die Vorrichtung gemäss Anspruch 1, wobei :
- die Vorrichtung ein Hybridschaltung-Plättchen (82) und wenigstens einen auf diesem Plättchen angebrachten Chip eines den(die) besagten RF-Telemetrie-Sender/Empfänger (44) integrierenden monolithischen Schaltkreises aufweist, und
- der BAW-Resonator (40) eine diskrete, vom Chip verschiedene, auf letzteren oberflächenmontierte Komponente ist.

9. Die Vorrichtung gemäss Anspruch 1, wobei :
- die Vorrichtung ein Hybridschaltung-Plättchen (82) und wenigstens einen auf diesem Plättchen angebrachten Chip eines den(die) besagten RF-Telemetrie-Sender/Empfänger (44) integrierenden monolithischen Schaltkreises aufweist, und
- der BAW-Resonator (40) eine diskrete, vom Chip verschiedene, auf dem Hybridschaltung-Plättchen oberflächenmontierte Komponente ist.

10. Die Vorrichtung gemäss Anspruch 1, in welcher der BAW-Resonator (40) ein abstimmbarer, eine erodierbare Oberfächenladungsschicht -74) aufweisender Resonator.

11. Die Vorrichtung gemäss Anspruch 1, in welcher die Bandsperre (54) ein eine Vielzahl von in Kreuzschaltung montierten BAW-Resonatoren (78,80) aufweisendes, einen differenzialem Eingang/Ausgang besitzendes Filter ist.

12. Die Vorrichtung gemäss Anspruch 1, in welcher die Bandsperre (54) ein eine Vielzahl von in L-Kettenchaltung montierten BAW-Resonatoren (78,80) aufweisendes, einen asymmetrischen Eingang/Ausgang besitzendes Filter ist.
